# EUROPÄISCHE PATENTSCHRIFT

(11) **EP 0 643 691 B1**
(45) Veröffentlichungstag und Bekanntmachung des Hinweises auf die Patenterteilung: **27.08.1997**
(21) Anmeldenummer: 93912774.2
(22) Anmeldetag: 24.05.1993
(51) Int. Cl.: C07C 305/10, C07C 303/24

(54) **VERFAHREN ZUR HERSTELLUNG GERUCHSARMER FETTALKOHOLETHERSULFAT-SALZE**
PROCESS FOR PREPARING ODOURLESS FATTY ALCOHOL ETHER SULPHATES
PROCEDE DE PREPARATION DE SULFATES D'ETHERS D'ALCOHOLS GRAS SANS ODEURS

(30) Priorität: 01.06.1992 DE 4218075
(43) Veröffentlichungstag der Anmeldung: 22.03.1995
(73) Patentinhaber: Henkel Kommanditgesellschaft auf Aktien, 40191 Düsseldorf (DE)
(72) Erfinder: BEHLER, Ansgar, D-46240 Bottrop (DE); PLOOG, Uwe, D-42781 Haan (DE); KÖHLER, Michael, D-40822 Mettmann (DE); HENSEN, Hermann, D-42781 Haan (DE); SEIPEL, Werner, D40723 Hilden (DE); DEMMERING, Günther, D-42653 Solingen (DE); KOMP, Horst-Dieter, D-40764 Langenfeld (DE)
(86) Internationale Anmeldenummer: EP9301300
(87) Internationale Veröffentlichungsnummer: WO9324453

(56) Entgegenhaltungen:
- EP-A- 0 337 406
- EP-A- 0 401 642
- AT-B- 353 761
- CH-A- 623 033
- DE-A- 3 933 860

## Beschreibung

Die Erfindung betrifft ein Verfahren zur Herstellung geruchsarmer Fettalkoholethersulfat-Salze, bei dem man ausgewählte Fettalkoholschnitte ethoxyliert, sulfatiert und anschließend neutralisiert.

### Stand der Technik

Anionische Tenside vom Typ der Fettalkoholethersulfate zeichnen sich durch starkes Schaumvermögen, hohe Reinigungskraft und geringe Härte- und Fettempfindlichkeit aus. Da sie zudem aus ökotoxikologischer Sicht unbedenklich sind, finden sie vielfach Verwendung zur Herstellung von Handgeschirrspülmitteln sowie kosmetischen Produkten wie beispielsweise Haarshampoos, Schaum- oder Duschbädern. Ein gewisser Nachteil, der mit der Verwendung von Fettalkoholethersulfaten verbunden ist, besteht in ihrem fettartigen Eigengeruch, der in der Regel eine Parfümierung erforderlich macht. Im Zuge geänderter Marktanforderungen besteht jedoch in letzter Zeit ein zunehmender Bedarf an schwach parfümierten oder parfümfreien Produkten und somit an geruchlich verbesserten Einsatzstoffen.

Obschon ein Ziel der DE-A1-3 933 860 ebenfalls darin besteht, geruchwarme Fettalkoholethersulfate zur Verfügung zu stellen, wird dies doch auf gänzlich anderem Wege erreicht. Die Lehre der DE-A1-3 933 860 besteht darin, Fettalkoholethoxylate mit einer sogenannten eingeengten Homologenverteilung und einer verminderten Menge freiem Fettalkohol in diese Sulfatierung einzusetzen.

Die Aufgabe der Erfindung bestand somit darin, ein Verfahren zur Herstellung geruchsarmer Ethersulfate zu entwickeln.

### Beschreibung der Erfindung

Gegenstand der Erfindung ist ein Verfahren zur Herstellung geruchsarmer Fettalkoholethersulfat-Salze der Formel (I),

**R**^{**1**}**-(OCH**_{**2**}**CH**_{**2**}**)**_{**n**}**-O-SO**_{**3**}**X** (I)

in der
- R¹: für einen Alkylrest mit 6 bis 18 Kohlenstoffatomen,
- n: für Zahlen von 1 bis 10 und
- X: für ein Alkali-, Erdalkali-, Ammonium- oder Alkanolammoniumion
steht,
durch Ethoxylierung von Fettalkoholen, Sulfatierung der resultierenden Fettalkoholethoxylate und nachfolgende Neutralisation der sauren Sulfierprodukte, das sich dadurch auszeichnet, daß man technische Fettalkoholschnitte einsetzt, deren Anteil an flüchtigen Verbindungen, die bei Normalbedingungen einen Siedepunkt unterhalb von 235°C aufweisen, maximal 0,5 Gew.-% - bezogen auf den Gesamtschnitt - beträgt.

Überraschenderweise wurde gefunden, daß Fettalkoholschnitte, die diese Anforderung erfüllen, nach Ethoxylierung und Sulfatierung Fettalkoholethersulfate ergeben, die praktisch geruchsfrei sind und die Marktbedürfnisse vollauf befriedigen. Die Erfindung schließt als Ergebnis umfangreicher analytischer Untersuchungen die Erkenntnis ein, daß alle geruchsverursachenden Nebenbestandteile technischer Fettalkohole, wie beispielweise kurzkettige Aldehyde, Ketone etc., bei Normalbedingungen einen Siedepunkt unterhalb von 235°C aufweisen.

Unter Normalbedingungen ist in diesem Zusammenhang eine Temperatur von 20°C und ein Außendruck von 1 bar (1 atm) zu verstehen.

**Fettalkoholethersulfate** stellen bekannte Tenside dar, die in an sich bekannter Weise durch Umsetzung von Fettalkoholen mit Ethylenoxid und nachfolgende Sulfatierung der resultierenden Fettalkoholethoxylate erhalten werden. Als besonders vorteilhaft hat sich das Verfahren für die Herstellung von geruchsarmen Fettalkoholethersulfaten der Formel (I) erwiesen, in der R¹ für einen Alkylrest mit 12 bis 14 Kohlenstoffen, n für Zahlen von 2 bis 5 und X für Natrium, Magnesium, Ammonium und/oder Alkanolammonium steht.

Die **Ethoxylierung** kann in Gegenwart von basischen Katalysatoren, wie beispielsweise Natriummethylat, oder Schichtverbindungen, wie beispielsweise hydrophobiertem Hydrotalcit, bei Temperaturen von 150 bis 190°C durchgeführt werden. Je nach verwendetem Katalysator können die resultierenden Fettalkoholethoxylate eine konventionelle oder auch eingeengte Homologenverteilung aufweisen.

Die **Sulfatierung** der Fettalkoholethoxylate kann mit Hilfe von gasförmigem Schwefeltrioxid oder vorzugsweise Chlorsulfonsäure erfolgen, wobei das molare Einsatzverhältnis zwischen Fettalkoholethoxylat und Sulfiermittel 1 : 0,9 bis 1,3, vorzugsweise 1 : 0,95 bis 1 : 1,05 betragen kann. Im Hinblick auf eine möglichst geringe Dioxanbelastung der Sulfatierungsprodukte hat es sich als vorteilhaft erwiesen, die Reaktion bei Temperaturen von 15 bis 50, vorzugsweise 25 bis 35°C durchzuführen und die Verweilzeit zwischen Sulfatierung und Neutralisation möglichst kurz zu gestalten. Die Neutralisation der sauren Produkte kann mit Hilfe wäßriger Basen, beispielsweise Natriumhydroxid, Kaliumhydroxid, Magnesiumhydroxid, Ammoniak oder Triethanolamin, durchgeführt werden.

Im Sinne des erfindungsgemäßen Verfahrens können vorteilhafterweise **technische Fettalkoholschnitte** eingesetzt werden, die folgende Zusammensetzung aufweisen:
=< C₁₀ = 0,1 bis 0,5 Gew.-%
C₁₂ = 20,0 bis 80,0 Gew.-%, insbesondere 50 bis 75 Gew.-%,
C₁₄ = 20,0 bis 80,0 Gew.-%, insbesondere 25 bis 35 Gew.-%,
>= C₁₆ = 0,5 bis 1,0

Fettalkoholschnitte dieser Art lassen sich beispielsweise erhalten, indem man technischen Kokosfett- oder Palmkernfettalkohol eines C-Kettenschnittes von C₈ bis C₁₈ in an sich bekannter Weise einer fraktionierten Destillation bzw.

Rektifikation unterwirft. Um eine scharfe Siedegrenze zu erhalten, die die Abtrennung von Stoffen mit einem Siedepunkt unterhalb von 235°C zuverlässig gewährleistet, kommen übliche technische Maßnahmen zur Trennschärfenverbesserung in Betracht. Hierzu zählt insbesondere die Verwendung von gepackten Kolonnen, die Wahl eines hohen Rücklaufverhältnisses sowie gegebenenfalls der Einsatz von Strippdampf zum Abtreiben von Schwersiedern.

### Gewerbliche Anwendbarkeit

Die nach dem erfindungsgemäßen Verfahren erhältlichen Fettalkoholethersulfat-Salze sind praktisch geruchsfrei. Sie eignen sich beispielsweise für die Herstellung schwach parfümierter oder parfümfreier Handgeschirrspülmittel sowie kosmetischer Produkte, wie Haarshampoos, Schaum- oder Duschbädern, in denen sie in Mengen von 1 bis 50, vorzugsweise 5 bis 35 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

a) **Ethoxylierung.** In einem 1,5-1-Stahlautoklaven wurden 835,1 g (4,3 mol) eines technischen Kokosfettalkoholdestillats der C-Kettenzusammensetzung
   C₁₀ : 0,3 Gew.-%
   C₁₂: 71,8 Gew.-%
   C₁₄: 26,6 Gew.-%
   C₁₆: 0,9 Gew.-%
   und 5,9 g Natriummethylat in Form einer 30 gew.- %igen Lösung in Methanol vorgelegt. Der Druckreaktor wurde mit Stickstoff gespült und 30 min bei 100°C evakuiert. Danach wurden bei einer Temperatur von 180°C portionsweise 350 g (8 mol) Ethylenoxid aufgedrückt, wobei sich ein Druck von 5 bar einstellte. Nach Abschluß der Ethylenoxidzugabe ließ man 30 min nachreagieren. Es wurden 1183 g Fettalkoholethoxylat (Hydroxylzahl : 205) erhalten.
b) **Sulfatierung.** In einem Vierhalskolben mit Rührer, Thermometer, Tropftrichter und Abgasstutzen wurden 276 g (1 mol) des Fettalkoholethoxylats aus Beispiel la) vorgelegt. Unter Rühren wurden 122,4 g (1,05 mol) Chlorsulfonsäure mit einer solchen Geschwindigkeit zugetropft, daß die Reaktionstemperatur 35°C nicht überschritt.
   Nach Beendigung der Reaktion wurde entstandenes HCl-Gas durch Evakuieren entfernt und das Rohprodukt mit wäßriger 25 gew.-%iger Natriumhydroxidlösung neutralisiert. Die Zusammensetzung des Produktes ist Tab.1 zu entnehmen.

### Vergleichsbeispiel V1:

a) **Ethozylierung.** Beispiel 1a) wurde unter Einsatz von technischem C_{12/14}-Kokosfettalkohol (Lorol^{(R)} S, Fa. Henkel KGaA, Düsseldorf, FRG) der C-Kettenzusammensetzung
   C₁₀ : 2,0 Gew.-%
   C₁₂: 70,0 Gew.-%
   C₁₄: 27,0 Gew.-%
   C₁₆: 1,0 Gew.-%
   wiederholt. Die Hydroxylzahl des Produktes betrug 205.
b) **Sulfatierung.** Analog Beispiel 1b) wurden 276 g des Fettalkoholethoxylats aus Beispiel 2a) mit Chlorsulfonsäure umgesetzt und neutralisiert. Die Zusammensetzung des Produktes ist Tab.1 zu entnehmen.

**Tab.1:**

| Zusammensetzung der Produkte Prozentangaben als Gew.-% | | | | | | |
|---|---|---|---|---|---|---|
| Bsp. | WAS % | US % | Na₂SO₄ % | NaCl % | H₂O % | Geruch |
| 1 | 26,9 | 0,6 | 0,9 | 0,10 | 70,7 | geruchsfrei |
| V1 | 25,6 | 1,3 | 1,0 | 0,04 | 71,3 | fettartig |
| Legende: WAS = Waschaktive Substanz (Aniontensidgehalt) US = Unsulfierte Substanz | | | | | | |

## Patentansprüche

1. Verfahren zur Herstellung geruchsarmer FettalkoholethersulfatSalze der Formel **(I)**,
**R**^{**1**}**-(OCH**_{**2**}**CH**_{**2**}**)**_{**n**}**-O-SO**_{**3**}**X (I)**
in der
R¹ für einen Alkylrest mit 6 bis 18 Kohlenstoffatomen,
n für Zahlen von 1 bis 10 und
X für ein Alkali-, Erdalkali-, Ammonium- oder Alkanolammoniumion
steht, durch Ethoxylierung von Fettalkoholen, Sulfatierung der resultierendem Fettalkoholethoxylate und nachfolgende Neutralisation der sauren Sulfierprodukte, **dadurch gekennzeichnet,** daß man technische Fettalkoholschnitte einsetzt, deren Anteil an flüchtigen Verbindungen, die bei Normalbedingungen einen Siedepunkt unterhalb von 235°C aufweisen, maximal 0,5 Gew.-% - bezogen auf den Gesamtschnitt - beträgt.

2. Verfahren nach Anspruch 1, **dadurch gekennzeichnet,** daß man technische Fettalkoholschnitte der folgenden Zusammensetzung einsetzt:
=< C₁₀ = 0,1 bis 0,5 Gew.-%
C₁₂ = 25,0 bis 75,0 Gew.-%
C₁₄ = 25,0 bis 75,0 Gew.-%
>= C₁₆ = 0,5 bis 1,0

## Claims

1. A process for the production of substantially odorless fatty alcohol ethersulfate salts corresponding to formula **(I)**:
**R**^{**1**}**-(OCH**_{**2**}**CH**_{**2**}**)**_{**n**}**-O-SO**_{**3**}**X** **(I)**
in which
R¹ is an alkyl radical containing 6 to 18 carbon atoms,
n is a number of 1 to 10 and
X is an alkali metal, alkaline earth metal, ammonium or alkanolammonium ion,
by ethoxylation of fatty alcohols, sulfation of the resulting fatty alcohol ethoxylates and subsequent neutralization of the acidic sulfonation products, **characterized in that** technical fatty alcohol cuts are used in which the percentage content of volatile compounds, which have a boiling point under normal conditions below 235°C, is at most 0.5% by weight, based on the cut as a whole.

2. A process as claimed in claim 1, **characterized in that** technical fatty alcohol cuts having the following composition are used:
=< C₁₀ = 0.1 to 0.5% by weight
C₁₂ = 25.0 to 75.0% by weight
C₁₄ = 25.0 to 75.0% by weight
>= C₁₆ = 0.5 to 1.0 % by weight

## Revendications

1. Procédé de préparation de sels d'éthersulfates d'alcools gras à faible odeur de formule **(I)**,
**R**^{**1**}**- (OCH**_{**2**}**CH**_{**2**}**)**_{**n**}**-O-SO**_{**3**}**X** **(I)**
où
R₁ est un radical alkyle de 6 à 18 atomes de carbone,
n représente un ion alcalin, alcalino-terreux, ammonium ou alcanolammonium,
par éthoxylation d'alcools gras, sulfatation des éthoxylates d'alcool gras résultants et neutralisation successive des produits de sulfatation acide,
caractérisé en ce qu'
on utilise des coupes d'alcools gras techniques dont la teneur en composés volatiles qui présentent dans les conditions normales un point d'ébullition inférieur à 235°C est au maximum 0,5 % en poids -par rapport à l'ensemble de la coupe-.

2. Procédé selon la revendication 1,
caractérisé en ce qu'
on utilise des coupes d'alcools gras techniques ayant la composition suivante :
≤ C₁₀ = 0,1 à 0,5 % en poids
C₁₂ = 25,0 à 75,0 % en poids
C₁₄ = 25,0 à 75,0 % en poids
≥ C₁₆ = 0,5 à 1,0 % en poids
